(19) Europäisches Patentamt
European Patent Office
Office européen des brevets

(11) EP 1 049 454 B1

(12) **EUROPÄISCHE PATENTSCHRIFT**

(45) Veröffentlichungstag und Bekanntmachung des Hinweises auf die Patenterteilung:
**19.03.2003 Patentblatt 2003/12**

(21) Anmeldenummer: **99907355.4**

(22) Anmeldetag: **07.01.1999**

(51) Int Cl.⁷: **A61K 7/48**

(86) Internationale Anmeldenummer:
**PCT/EP99/00054**

(87) Internationale Veröffentlichungsnummer:
**WO 99/037273 (29.07.1999 Gazette 1999/30)**

(54) **KOSMETISCHE UND DERMATOLOGISCHE ZUBEREITUNGEN, ENTHALTEND ERHÖHTE ELEKTROLYTKONZENTRATIONEN**

COSMETIC AND DERMATOLOGICAL PREPARATIONS CONTAINING HIGHER ELECTROLYTE CONCENTRATIONS

PREPARATIONS COSMETIQUES ET DERMATOLOGIQUES CONTENANT DES CONCENTRATIONS D'ELECTROLYTE ACCRUES

(84) Benannte Vertragsstaaten:
**DE ES FR GB IT SE**

(30) Priorität: **22.01.1998 DE 19802205**

(43) Veröffentlichungstag der Anmeldung:
**08.11.2000 Patentblatt 2000/45**

(73) Patentinhaber: **Beiersdorf Aktiengesellschaft
20245 Hamburg (DE)**

(72) Erfinder:
- **BLECKMANN, Andreas
D-22926 Ahrensburg (DE)**
- **VON DER FECHT, Stephanie
D-22869 Schenefeld (DE)**
- **HAMER, Gunhild
D-22455 Hamburg (DE)**
- **SCHNEIDER, Günther
D-22607 Hamburg (DE)**

(56) Entgegenhaltungen:
**EP-A- 0 801 944** **EP-A- 0 808 842**
**GB-A- 1 210 022** **US-A- 4 427 671**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 049 454 B1

## Beschreibung

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, die Stabilität von elektrolythaltigen Zubereitungen, insbesondere Emulsionen, bevorzugt von O/W-Emulsionen, zu steigern.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muß deshalb ununterbrochen erneuert werden.

Ein heute in der Fachwelt weitverbreitetes Hautmodell faßt das Stratum corneum als Zwei-Komponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig.

Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert. Damit formulierte Produkte unterstützen die flüssigkristalline Organisation der Interzellularlipide des Stratum Comeums und verbessern so die Barriereeigenschaften der Homschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautatterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase ) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gellnetzwerden zurückgeführt.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche

Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-EMulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können.

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es manchmal zweckmäßiger für die Schilderung der vorliegenden Erfindung und ihres technischen Hintergrundes, von der lonenstärke eines gegebenen Elektrolytes in seiner Lösung auszugehen.

Die lonenstärke *I* einer Elektrolytiösung ist definiert als

$$I = \tfrac{1}{2} \sum_i c_i z_i^2$$

wobei $c_i$ die Konzentrationen der einzelnen lonensorten (in mol/l) und $z_i$ deren Ladungszahlen darstellen. Die physikalische Einheit der lonenstärke ist die einer Konzentration (mol/l).

Eine 1-%ige ( = 0,17-molare) Kochsalzlösung hat beispielsweise eine lonenstärke *I* = 0,17.

Eine weitere Aufgabe der vorliegenden Erfindung war es also, Lösungswege zu kosmetischen oder dermatologischen Emulsionen, insbesondere O/W-Emutsionen. aufzudecken, welche gegenüber erhöhten Elektrolytkonzentrationen - bzw. erhöhten Ionenstärken - stabil sind.

Ferner war eine Aufgabe der vorliegenden Erfindung, Zubereitungen zur Verfügung zu stellen, welche den Zustand der Haut deutlich verbessern, insbesondere die Hautrauhigkeit vermindern.

Es hat sich überraschend gezeigt, und darin liegt die Lösung dieser Aufgaben, daß kosmetische und dermatologische Zubereitungen mit mindestens einer wäßrigen Phase, enthaltend

(1) einen oder mehrere partiell neutralisierte Ester von Monoglyceriden und/oder Diglyceriden gesättigter Fettsäuren mit Zitronensäure,

(II) einen oder mehrere Sorbitanmonoester, welche sich durch die Strukturformel

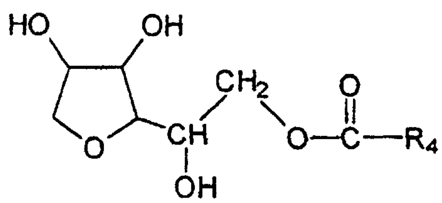

auszeichnen, wobei $R_4$ einen verzweigten oder unverzweigten Alkylrest mit 8 bis 22 Kohlenstoffatomen darstellt.

(III) einen oder mehrere Fettalkohole, gewählt aus der Gruppe der verzweigten und unverzweigten Alkylalkohole mit 12 bis 40 Kohlenstoffatomen,

(IV) wobei mindestens eine der wäßrigen Phasen einen oder mehrere Elektrolyte gelöst enthält,

den Nachteilen des Standes der Technik abhelfen.

Es war für den Fachmann daher nicht vorauszusehen gewesen, daß die erfindungsgemäßen Zubereitungen

- besser als feuchtigkeitsspendende Zubereitungen wirken,
- besser die Hautglättung fördern,
- sich durch besser Pflegewirkung auszeichen,
- besser als Vehikel für kosmetische und medizinisch-dermatologische Wirkstoffe dienen
- höhere Stabilität gegenüber Zerfall in Öl- und Wasserphasen aufweisen und
- sich durch bessere Bioverträglichkeit auszeichnen würden

als die Zubereitungen des Standes der Technik.

Erfindungsgemäß ist ferner auch die Verwendung von kosmetischen und dermatologischen Emulsionen, insbesondere O/W-Emulsionen mit mindestens einer wäßrigen Phase, enthaltend

(I) einen oder mehrere partiell neutralisierte Ester von Monoglyceriden und/oder Diglyceriden gesättigter Fettsäuren mit Zitronensäure,

(II) einen oder mehrere Sorbitanmonoester, welche sich durch die Strukturformel

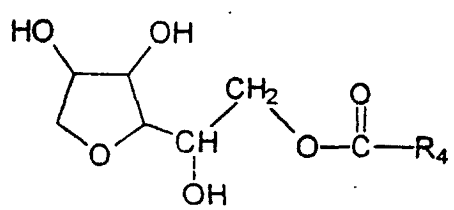

auszeichnen, wobei $R_4$ einen verzweigten oder unverzweigten Alkylrest mit 8 bis 22 Kohlenstoffatomen darstellt.

(III) einen oder mehrere Fettalkohole, gewählt aus der Gruppe der verzweigten und unverzweigten Alkylalkohole mit 12 bis 40 Kohlenstoffatomen,

(IV) wobei mindestens eine der wäßrigen Phasen einen oder mehrere Elektrolyte gelöst enthält,

zur Herstellung von kometischen und dermatologischen Emulsionen, insbesondere O/W Emulsion mit mindestens einer wäßrigen Phase, zur Pflege der Haut.

Erfindungsgemäß ist weiterhin auch die Verwendung der Kombination aus

(I) einem oder mehreren partiell neutralisierten Estem von Monoglyceriden und/oder Diglyceriden gesättigter Fettsäuren mit Zitronensäure,

(II) einem oder mehreren Sorbitanmonoestern, welche sich durch die Strukturformel

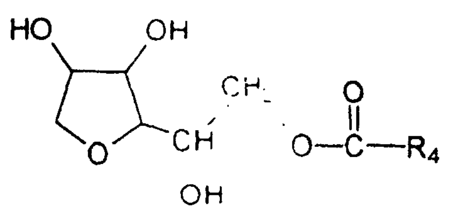

auszeichnen, wobei $R_4$ einen verzweigten oder unverzweigten Alkylrest mit 8 bis 22 Kohlenstoffatomen darstellt.

(III) einem oder mehreren Fettalkoholen, gewahlt aus der Gruppe der verzweigten und unverzweigten Alkylalkohole mit 12 bis 40 Kohlenstoffatomen zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten.

Erfindungsgemäß ist insbesondere die Verwendung der Kombination aus

(I) einen oder mehrere partiell neutralisierte Ester von Monoglyceriden und/oder Diglyceriden gesättigter Fettsäuren mit Zitronensäure,

(II) einen oder mehrere Sorbitanmonoester, welche sich durch die Strukturformel

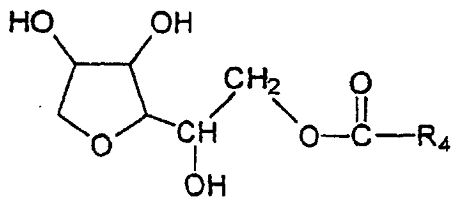

auszeichnen, wobei $R_4$ einen verzweigten oder unverzweigten Alkylrest mit 8 bis 22 Kohlenstoffatomen darstellt.
(III) einen oder mehrere Fettalkohole gewählt aus der Gruppe der verzweigten und unverzweigten Alkylalkohole mit 12 bis 40 Kohlenstoffatomen

zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wäßrigen Phasen einen oder mehrere Elektrolyte gelöst enthält, und die Konzentration der in Ionen dissoziierten Substanzen in der oder den wäßrigen Phasen, in der der oder die Elektrolyte gelöst vorliegen, mindestens 0,05 mol/l, insbesondere mindestens 0,075 mol/l, besonders bevorzugt mindestens 0,10 mol/l, beträgt.

Erfindungsgemäß ist insbesondere die Verwendung der Kombination aus

(I) einen oder mehrere partiell neutralisierte Ester von Monoglyceriden und/oder Diglyceriden gesättigter Fettsäuren mit Zitronensäure,
(II) einen oder mehrere Sorbitanmonoester, welche sich durch die Strukturformel

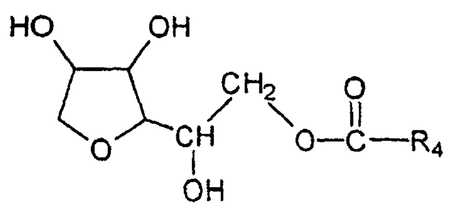

auszeichnen, wobei $R_4$ einen verzweigten oder unverzweigten Alkylrest mit 8 bis 22 Kohlenstoffatomen darstellt.
(III) einen oder mehrere Fettalkohole gewählt aus der Gruppe der verzweigten und unverzweigten Alkylalkohole mit 12 bis 40 Kohlenstoffatomen

zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wäßrigen Phasen einen oder mehrere Elektrolyte gelöst enthält, und die Ionenstärke der wäßrigen Phasen, in der der oder die Elektrolyte gelöst vorliegen, mindestens 0,05 mol/l, insbesondere mindestens 0,075 mol/l, besonders bevorzugt mindestens 0,10 mol/l beträgt.

Ein besonders vorteilhafter Citronensäureester ist das Glycerylstearatcitrat. Solche Citronensäureester sind beispielsweise erhältlich unter der Produktbezeichnung "IMWI- TOR® 370" der Gesellschaft Hüls AG.

Die Gesamtmenge an einem oder mehreren erfindungsgemäß verwendeten Glycerinestern von α-Hydroxycarbonsäuren und gesättigten Fettsäuren in den fertigen kosmetischen oder dermatologischen Zubereitungen wird vorteilhaft aus dem Bereich von 0,1 - 10,0 Gew.-%, bevorzugt 0,5-6,0 Gew.-% gewählt, bezogen auf das Gesamtgewicht der Zubereitungen.

Besonders vorteilhaft wird als grenzflächenaktive Substanz aus der Gruppe der Sorbitanmonoester das Sorbitanmonstearat gewählt, welches sich durch die Strukturformel

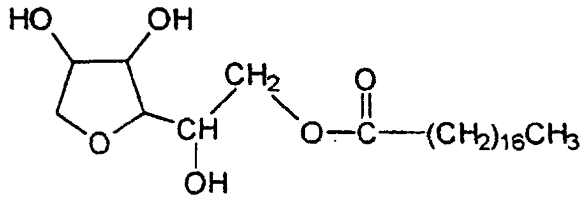

auszeichnet. Ein solches Produkt ist im Handel beispielsweise unter der Warenbezeichnung Arlacel® 60 (INCI: Sorbitan Stearate) von der Gesellschaft ICI erhältlich.

Die Gesamtmenge an einer oder mehreren erfindungsgemäß verwendeten grenzflächenaktiven Sorbitanestern in den fertigen kosmetischen oder dermatologischen Zubereitungen wird vorteilhaft aus dem Bereich von 0,1 - 25,0 Gew.-%, bevorzugt 0,5 - 15,0 Gew.-% gewählt, bezogen auf das Gesamtgewicht der Zubereitungen.

Bevorzugter, erfindungsgemäß verwendeter Fettalkohol ist der Cetyl-Stearylalkohol (ein Gemisch aus Hexadecanol-1 und Octadecanol-1 zu etwa gleichen Anteilen).

Die Gesamtmenge an einem oder mehreren erfindungsgemäß verwendeten Fettalkoholen in den fertigen kosmetischen oder dermatologischen Zubereitungen wird vorteilhaft aus dem Bereich von 0,1 - 10,0 Gew.-%, bevorzugt 0,5 -6,0 Gew.-% gewählt, bezogen auf das Gesamtgewicht der Zubereitungen.

Es ist erfindungsgemäß vorteilhaft, Gewichtsverhältnisse von partiell neutralisierten Estern von Monoglyceriden und/oder Diglyceriden gesättigter Speisefettsäuren mit Zitronensäure (Glycerylstearatcitrat, nach INCI: Glyceryl Stearate Citrate) und Sorbitanestern einerseits und einen oder mehrere Fettalkohole (gewählt aus der Gruppe der verzweigten und unverzweigten Alkylalkohole mit 12 bis 40 Kohlenstoffatomen) andererseits von 7 : 3 bis 3 : 7 zu wählen, bevorzugt von 2 : 1 bis 1 : 2, insbesondere bevorzugt von etwa 1 : 1.

Bevorzugt stellen die erfindungsgemäßen Zubereitungen Emulsionen, insbesondere O/W-Emulsionen dar.

Erfindungsgemäß werden der oder die Elektrolyte vorteilhaft gewählt aüs der Gruppe

(1) der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

(1a) Harnstoff in Kombination mit Natriumlactat / Milchsäure bzw. Natriumcitrat / Citronensäure.

Erfindungsgemäß werden der oder die Elektrolyte ferner vorteilhaft gewählt aus der Gruppe

(2) Bestimmte wasserlösliche, zumeist als Alkalisalze vorliegende wasserlösliche UV-Filtersubstanzen, insbesondere solche, die an ihrem Molekülgerüst eine oder mehrere Sulfonsäuregruppen bzw. Sulfonatgruppen tragen:

Die 2-Phenylbenzimidazol-5-sulfonsäure und ihre Salze, beispielsweise das Natrium-, Kalium- oder ihr Triethanolammonium-Salz

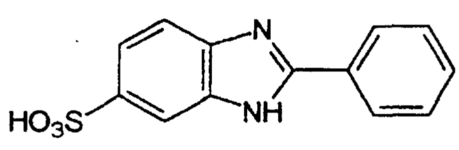

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

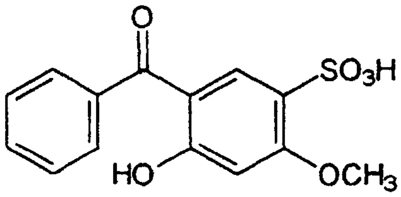

Sutfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

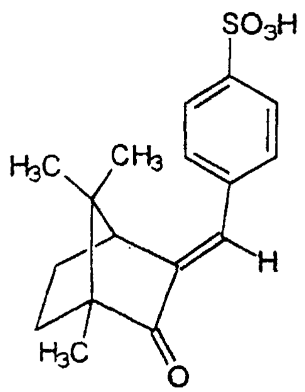

Die 2-Methyl-5-(2-oxo-3-bornylidenmethyl)benzolsulfonsäure und ihre Salze, beispielsweise das entsprechende Natrium-, Kalium- oder Triethanolammonium-Salz:

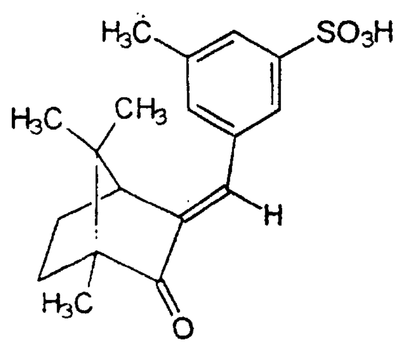

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

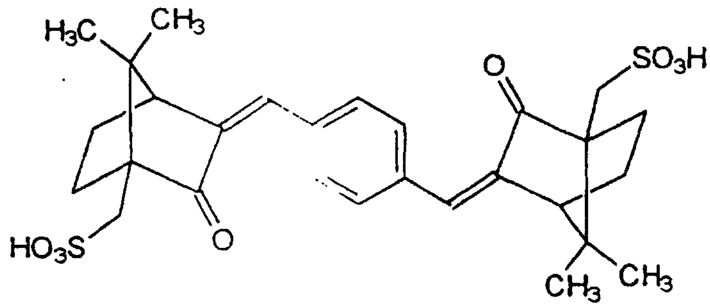

Erfindungsgemäß werden der oder die Elektrolyte weiterhin vorteilhaft gewählt aus der Gruppe
(3) der Aminosäuren und deren Salze bzw deren Anionen. Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydratationsvorgänge Feuchtigkeit in der Haut gebunden werden kann.

Aminosäuren mit besonders vorteilhafter kosmetischer bzw. dermatologischer Wirkung sind Glycin, Alanin, Valin, Leucin, Isoleucin, Phenylalanin, Tyrosin, Prolin, Hydroxyprolin, Serin, Threonin, Cystein. Methionin, Tryptophan, Arginin.

Erfindungsgemäß werden der oder die Elektrolyte weiter vorteilhaft gewählt aus der Gruppe

(4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen.

α-Hydroxycarbonsäuren, welche kosmetisch oder dermatologisch relevant sind, folgen der allgemeinen Formel

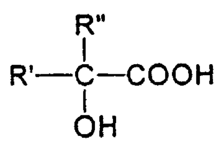

β-Hydroxycarbonsäuren, welche kosmetisch oder dermatologisch relevant sind, folgen der allgemeinen Formel

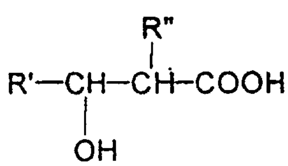

bzw.

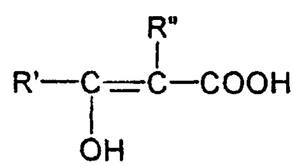

α-Ketocarbonsäuren, welche kosmetisch oder dermatologisch relevant sind, folgen der allgemeinen Formel

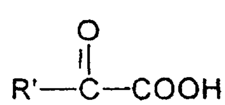

wobei jeweils R' und R" unabhängig voneinander gewählt werden aus der Gruppe

(a1) H-,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(a4) Phenyl-,
(a5) mit einer oder mehreren Carboxylgruppen und/oder Hydroxygruppen und/oder verzweigten und/oder unverzweigten $C_{1-25}$-Alkylgruppen substituiertes Phenyl-,
oder wobei das α-Kohlenstoffatom und das β-Kohienstoffatom der β-Hydroxycarbonsäure mit R' und R" zusammen eine
(a6) unsubstituierte Cycloalkylgruppe oder Arylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen ausbildet und

wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:

Die Salicylsäure (auch 2-Hydroxybenzoësäure, Spirsäure), welche durch die Struktur

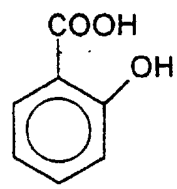

gekennzeichnet ist. Bekanntermaßen wirkt Salicylsäure antibakteriell und keratolytisch und ist Bestandteil mancher kosmetischen oder pharmazeutischen Zubereitungen.

Die den erfindungsgemäß verwendeten α-Hydroxycarbonsäuren werden vorteilhaft gewählt aus folgenden Substanzklassen:

(a2) α-Hydroxyfettsäuren, wobei diese wiederum besonders vorteilhaft aus der Gruppe der $C_{10-18}$-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe

- α-Hydroxycarbonsäuren, gemäß der Formel

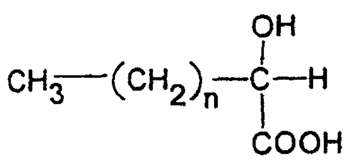

und/oder
- α-Hydroxy-isocarbonsäuren, gemäß der Formel

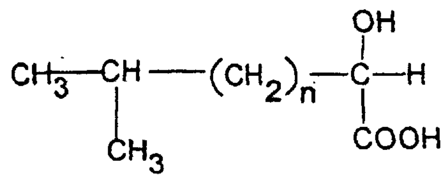

und/oder

- α-Hydroxy-anteisocarbonsäuren, gemäß der Formel

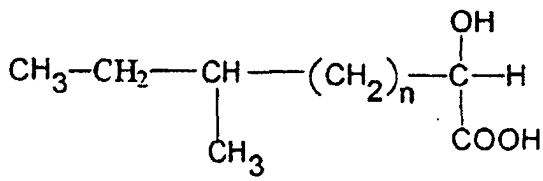

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

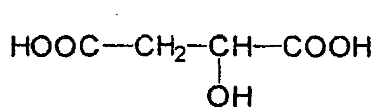

Milchsäure (2-Hydroxypropansäure) ist durch folgende chemische Struktur gekennzeichnet:

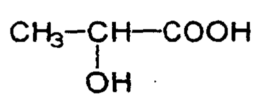

Citronensäure (2-Hydroxy-1,2,3-propantricarbonsäure) ist durch folgende chemische Struktur gekennzeichnet:

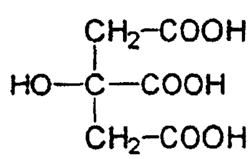

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet:

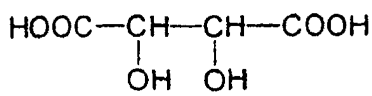

Bevorzugte α-Ketocarbonsäure ist die Brenztraubensäure (α-Oxopropansäure). Sie zeichnet sich durch folgende Struktur aus:

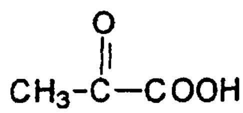

Die Höchstmenge der einzusetztenden Elektrolyte ist letztendlich abhängig von deren Löslichlichkeit in der wäßrigen Phase. Grundsetzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolytes hinausgehende zusätzliche Menge dieses Elektrolytes, beispielsweise als ungelösten Festkörper, in eine kosmetische oder dermatologische Zubereitung einzuarbeiten.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird, n und m sind dabei nach Belieben einzusetzende stöchiometrische Parameter, R und R' die gewünschten organischen Reste, Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin. Tyrosin, Tryptophan) und deren Derivate. Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat). Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%. bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter

und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl. Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cydischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%. insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemaßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 20 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäHarnstoffmylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind bereits unter dem Begriff der erfindungsgemäß vorteilhaften Elektrolyte genannt worden, seien hier aber noch einmal auszugsweise genannt:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Es ist auch besonders vorteilhaft, die erfindungsgemäßen Wirkstoffkombinationen mit Salicylsäurederivaten zu kombinieren, von denen einige Vertreter bekannt sind, welche ebenfalls UV-Strahlung absorbieren können. Zu gebräuchlichen UV-Filtern gehören

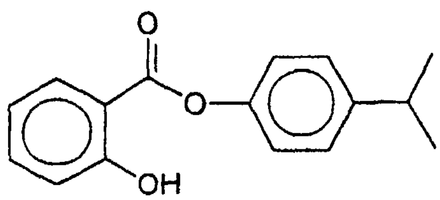

**(4-Isopropylbenzylsalicylat),**

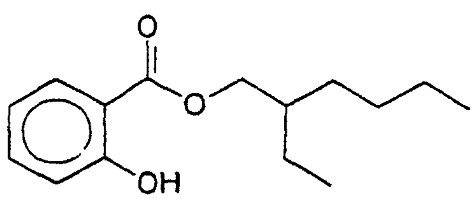

**(2-Ethylhexylsalicylat, Octylsalicylat),**

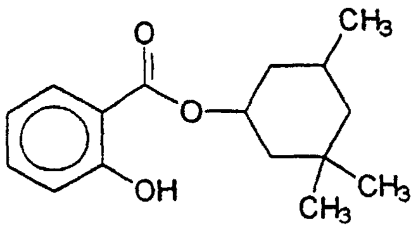

**(Homomenthylsalicylat).**

Die Herstellung der erfindungsgemäßen kosmetischen und/oder dermatologischen Zubereitungen erfolgt in der dem Fachmann üblichen Weise, zumeist dergestallt, daß eine Ölphase mit einem Gehalt an erfindungsgemäß verwendeten grenzflächenaktiven Glucosederivaten bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wäßrigen Phase, in welche

gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1 (O/W-Emulsion):** | |
|---|---|
| | Gew.-% |
| Glycerylstearatcitrat | 3,60 |
| Sorbitanmonostearat | 1,20 |
| Cetylstearylalkohol | 1,20 |
| Octytdodecanol | 3,00 |
| Caprylic/Capric Triglyceride | - 3,00 |
| Dicaprylylether | 3,00 |
| Xanthangummi | 0,10 |
| Glycerin | 3,00 |
| Milchsäure (90%) | 0,25 |
| Natriumlactat (50%) | 7,50 |
| Parfüm, Konservierungsmittel, Farbstoffe, Antioxidantien usw. | q.s. |
| Wasser | ad 100,00 |
| pH-Wert eingestellt auf | 5,0 |

| **Beispiel 2 (O/W-Emulsion):** | |
|---|---|
| | Gew.-% |
| Glycerylstearatcitrat | 2,40 |
| Sorbitanmonostearat | 0,80 |
| Cetylstearylalkohol | 0,80 |
| Octyldodecanol | 3,00 |
| Caprylic/Capric Triglyceride | 3,00 |
| Dicaprylylether | 3,00 |
| Xanthangummi | 0,10 |
| Carbomer | 0,10 |
| Glycerin | 3,00 |
| Milchsäure (90%) | 0,25 |
| Natriumlactat (50%) | 7,50 |
| Parfüm, Konservierungsmittel, NaOH Farbstoffe, Antioxidantien usw. | q.s. |
| Wasser | ad 100,00 |
| pH-Wert eingestellt auf | 5,0 |

| **Beispiel 3 (O/W-Emulsion):** | |
|---|---|
| | Gew.-% |
| Glycerylstearatcitrat | 3,60 |
| Sorbitanmonostearat | 1,20 |
| Cetylstearylalkohol | 1,20 |
| Octyldodecanol | 3,00 |
| Caprylic/Capric Triglyceride | 3,00 |
| Dicaprylylether | 3,00 |

(fortgesetzt)

| Beispiel 3 (O/W-Emulsion): | |
|---|---|
| | Gew.-% |
| Xanthangummi | 0,10 |
| Glycerin | 3,00 |
| Citronensäure | 0,09 |
| Natriumcitrat | 0,18 |
| Parfüm, Konservierungsmittel, Farbstoffe, Antioxidantien usw. | q.s. |
| Wasser | ad 100,00 |
| pH-Wert eingestellt auf | 5,0 |

| Beispiel 4 (O/W-Emulsion): | |
|---|---|
| | Gew.-% |
| Glycerylstearatcitrat | 3,60 |
| Sorbitanmonostearat | 1,20 |
| Cetylstearylalkohol | 1,20 |
| Octyldodecanol | 3,00 |
| Caprylic/Capric Triglyceride | 1,00 |
| Squalan | 2,00 |
| Jojoba Öl | 1,00 |
| Cyclomethicon | 1,00 |
| Dimethicon | 0,50 |
| Paraffinum liquidum | 1,00 |
| Hydrierte Kokosfettsäureglyceride | 2,00 |
| Xanthangummi | 0,10 |
| Glycerin | 3,00 |
| Milchsäure (90%) | 0,25 |
| Natriumlactat (50%) | 7,50 |
| Tocopherolacetat | 1,00 |
| Serin | 0,50% |
| Parfüm, Konservierungsmittel, Farbstoffe, Antioxidantien usw. | q.s. |
| Wasser | ad 100,00 |
| pH-Wert eingestellt auf | 5,0 |

| Beispiel 5 (O/W-Emulsion): | |
|---|---|
| | Gew.-% |
| Glycerylstearatcitrat | 2,50 |
| Sorbitanmonostearat | 1,50 |
| Cetylstearylalkohol | 1,00 |
| Octyldodecanol | 3,00 |
| Caprylic/Capric Triglyceride | 3,00 |
| Dicaprylylether | 3,00 |
| Xanthangummi | 0,10 |
| Glycerin | 3,00 |
| Milchsäure (90%) | 0,25 |
| Natriumlactat (50%) | 7,50 |
| Parfüm, Konservierungsmittel, Farbstoffe, Antioxidantien usw. | q.s. |
| Wasser | ad 100,00 |

(fortgesetzt)

| Beispiel 5 (O/W-Emulsion): | |
|---|---|
| | Gew.-% |
| pH-Wert eingestellt auf | 5,0 |

| Beispiel 6 (O/W-Emulsion): | |
|---|---|
| | Gew.-% |
| Glycerylstearatcitrat | 3,00 |
| Sorbitanmonostearat | 1,00 |
| Cetylstearylalkohol | 1,50 |
| Octyldodecanol | 3,00 |
| Caprylic/Capric Triglyceride | 3,00 |
| Dicaprylylether | 3,00 |
| Xanthangummi | 0,10 |
| Carbomer | 0,10 |
| Glycerin | 3,00 |
| Natriumchlorid | 0,30% |
| Parfüm, Konservierungsmittel, Farbstoffe, Antioxidantien usw. | q.s. |
| Wasser | ad 100,00 |
| pH-Wert eingestellt auf | 5,5 |

| Beispiel 7 (O/W-Emulsion): | |
|---|---|
| | Gew.-% |
| Glycerylstearatcitrat | 3,00 |
| Sorbitanmonostearat | 1,00 |
| Cetylstearylalkohol | 1,00 |
| Octyldodecanol | 3,00 |
| Caprylic/Capric Triglyceride | 3,00 |
| Dicaprylylether | 3,00 |
| Xanthangummi | 0,10 |
| Carbomer | 0,10 |
| Glycerin | 3,00 |
| Arginin Hydrochloride | 1,20 |
| Parfüm, Konservierungsmittel, Farbstoffe, Antioxidantien usw. | q.s. |
| Wasser | ad 100,00 |
| pH-Wert eingestellt auf | 5,0 |

| Beispiel 8 (O/W-Emulsion): | |
|---|---|
| | Gew.-% |
| Glycerylstearatcitrat | 3,60 |
| Sorbitanmonostearat | 1,20 |
| Cetylstearylalkohol | 1,20 |
| Octyldodecanol | 3,00 |
| Caprylic/Capric Triglyceride | 3,00 |
| Dicaprytylether | 3,00 |
| Xanthangummi | 0,10 |

(fortgesetzt)

| Beispiel 8 (O/W-Emulsion): | |
|---|---|
| | Gew.-% |
| Carbomer | 0,10 |
| Glycerin | 3,00 |
| Milchsäure (90%) | 2,00 |
| Parfüm, Konservierungsmittel, NaOH Farbstoffe, Antioxidantien usw. | q.s. |
| Wasser | ad 100,00 |
| pH-Wert eingestellt auf | 4,0 |

| Beispiel 9 (O/W-Emulsion): | |
|---|---|
| | Gew.-% |
| Glycerytstearatcitrat | 3,00 |
| Sorbitanmonostearat | 1,00 |
| Cetylstearylalkohol | 1,00 |
| Octyldodecanol | 3,00 |
| Caprylic/Capric Triglyceride | 3,00 |
| Dicaprylylether | 3,00 |
| Xanthangummi | 0,10 |
| Carbomer | 0,10 |
| Glycerin | 3,00 |
| Milchsäure (90%) | 0,12 |
| Natriumlactat (50%) | 8,00 |
| Harnstoff | 4,00 |
| Octylmethoxycinnamat | 4,00 |
| Benzophenone-3 | 3,00 |
| Octylsalicylat | 3,00 |
| Parfüm, Konservierungsmittel Farbstoffe, Antioxidantien usw. | q.s. |
| Wasser | ad 100,00 |
| pH-Wert eingestellt auf | 5,5 |

| Beispiel 10 (Emulgatorgel): | |
|---|---|
| | Gew.-% |
| Glycerylstearatcitrat | 3,60 |
| Sorbitanmonostearat | 1,20 |
| Cetylstearylalkohol | 1,20 |
| Xanthangummi | 0,10 |
| Glycerin | 3,00 |
| Milchsäure (90%) | 0,25 |
| Natriumlactat (50%) | 7,50 |
| Parfüm, Konservierungsmittel, Farbstoffe, Antioxidantien usw. | q.s. |
| Wasser | ad 100,00 |
| pH-Wert eingestellt auf | 5,0 |

| Beispiel 11 (Deo): | |
|---|---|
| | Gew.-% |
| Glycerylstearatcitrat | 3,60 |
| Sorbitanmonostearat | 1,20 |
| Cetylstearylalkohol | 1,20 |
| Xanthangummi | 0,10 |
| Glycerin | 3,00 |
| Ethanol | 10,00 |
| Aluminiumchlorohydrat | 3,00 |
| Parfüm, Konservierungsmittel, Farbstoffe, Antioxidantien usw. | q.s. |
| Wasser | ad 100,00 |
| pH-Wert eingestellt auf | 4,5 |

**Patentansprüche**

**1.** Kosmetische und dermatologische Zubereitungen mit mindestens einer wäßrigen Phase, enthaltend

(I) einen oder mehrere partiell neutralisierte Ester von Monoglyceriden und/oder Diglyceriden gesättigter Fettsäuren mit Zitronensäure,
(II) einen oder mehrere Sorbitanmonoester, welche sich durch die Strukturformel

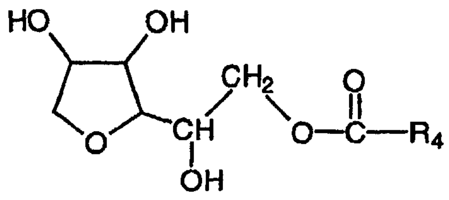

auszeichnen, wobei $R_4$ einen verzweigten oder unverzweigten Alkylrest mit 8 bis 22 Kohlenstoffatomen darstellt.
(III) einen oder mehrere Fettalkohole, gewählt aus der Gruppe der verzweigten und unverzweigten Alkylalkohole mit 12 bis 40 Kohlenstoffatomen,
(IV) wobei mindestens eine der wäßrigen Phasen einen oder mehrere Elektrolyte gelöst enthält.

**2.** Verwendung von

(I) einen oder mehrere partiell neutralisierte Ester von Monoglyceriden und/oder Diglyceriden gesättigter Fettsäuren mit Zitronensäure,
(II) einen oder mehrere Sorbitanmonoester, welche sich durch die Strukturformel

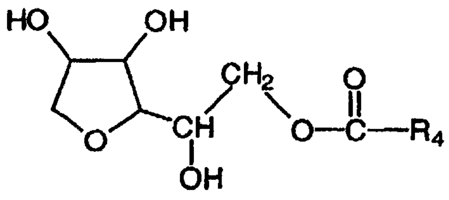

auszeichnen, wobei $R_4$ einen verzweigten oder unverzweigten Alkylrest mit 8 bis 22 Kohlenstoffatomen darstellt.
(III) einen oder mehrere Fettalkohole, gewählt aus der Gruppe der verzweigten und unverzweigten Alkylalkohole mit 12 bis 40 Kohlenstoffatomen,
(IV) wobei mindestens eine der wäßrigen Phasen einen oder mehrere Elektrolyte gelöst enthält,

zur Herstellung von kosmetischen und dermatologischen Emulsionen, insbesondere O/W-Emulsionen mit mindestens einer wäßrigen Phase, zur Pflege der Haut.

**3.** Verwendung der Kombination aus

(I) einem oder mehreren partiell neutralisierten Estern von Monoglyceriden und/oder Diglyceriden gesättigter Fettsäuren mit Zitronensäure,
(II) einem oder mehreren Sorbitanmonoestem, welche sich durch die Strukturformel

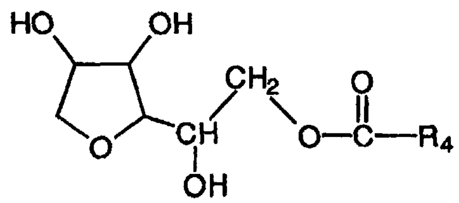

auszeichnen, wobei $R_4$ einen verzweigten oder unverzweigten Alkylrest mit 8 bis 22 Kohlenstoffatomen darstellt.
(III) einem oder mehreren Fettalkoholen, gewählt aus der Gruppe der verzweigten und unverzweigten Alkylalkohole mit 12 bis 40 Kohlenstoffatomen,

zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten.

**4.** Verwendung nach Anspruch 3 zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wäßrigen Phasen einen oder mehrere Elektrolyte gelöst enthält, und die Konzentration der in Ionen dissoziierten Substanzen in der oder den wäßrigen Phasen, in der der oder die Elektrolyte gelöst vorliegen, mindestens 0,05 mol/l, insbesondere mindestens 0,075 mol/l, besonders bevorzugt mindestens 0,10 mol/l, beträgt.

**5.** Verwendung nach Anspruch 3 zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wäßrigen Phasen einen oder mehrere Elektrolyte gelöst enthält, und die lonenstärke der wäßrigen Phasen, in der der oder die Elektrolyte gelöst vorliegen, mindestens 0,05 mol/l, insbesondere mindestens 0,075 mol/l, besonders bevorzugt mindestens 0,10 mol/l beträgt.

**6.** Zubereitung nach Anspruch 1, **dadurch gekennzeichnet, daß** die Gesamtmenge an einem oder mehreren partiell neutralisierten Estern von Monoglyceriden und/oder Diglyceriden gesättigter Fettsäuren mit Zitronensäure in den fertigen kosmetischen oder dermatologischen Zubereitungen gewählt wird aus dem Bereich von 0,1 - 10,0 Gew.-%, bevorzugt 0,5 - 6,0 Gew.-%, bezogen auf das Gesamtgewicht der Zubereitungen.

**7.** Zubereitung nach Anspruch 1, **dadurch gekennzeichnet, daß** die Gesamtmenge an einem oder mehreren grenzflächenaktiven Substanzen aus der Gruppe der Sorbitanmonoester gewählt wird aus dem Bereich von 0,1 - 25,0 Gew.-%, bevorzugt 0,5 - 15,0 Gew.-%, bezogen auf das Gesamtgewicht der Zubereitungen.

**8.** Zubereitung nach Anspruch 1, **dadurch gekennzeichnet, daß** die Gesamtmenge an einem oder mehreren Fettalkoholen in den fertigen kosmetischen oder dermatologischen Zubereitungen gewählt wird aus dem Bereich von 0,1- 10,0 Gew.-%, bevorzugt 0,5 - 6,0 Gew.-%, bezogen auf das Gesamtgewicht der Zubereitungen.

**9.** Zubereitung nach Anspruch 1, **dadurch gekennzeichnet, daß** Gewichtsverhältnisse von partiell neutralisierten Estern von Monoglyceriden und/oder Diglyceriden gesättigter Speisefettsäuren mit Zitronensäure und Sorbitanestern einerseits und einen oder mehrere Fettalkohole (gewählt aus der Gruppe der verzweigten und unverzweigten Alkylalkohole mit 12 bis 40 Kohlenstoffatomen) andererseits von 7 : 3 bis 3 : 7 gewählt werden, bevorzugt von 2: 1 bis 1 : 2, insbesondere bevorzugt von etwa 1:1.

**10.** Zubereitung nach Anspruch 1, **dadurch gekennzeichnet, daß** als partiell neutralisierten Estern von Monoglyceriden und/oder Diglyceriden gesättigter Speisefettsäuren mit Zitronensäure das Glycerylstearatcitrat gewählt wird.

**11.** Verwendung nach einem der Ansprüche 2 - 5, **dadurch gekennzeichnet, daß** die Gesamtmenge an einem oder mehreren partiell neutralisierten Estern von Monoglyceriden und/oder Diglyceriden gesättigter Fettsäuren mit Zitronensäure in den fertigen kosmetischen oder dermatologischen Zubereitungen gewählt wird aus dem Bereich von 0,1 - 10,0 Gew.-%, bevorzugt 0,5 - 6,0 Gew.-%, bezogen auf das Gesamtgewicht der Zubereitungen.

**12.** Verwendung nach einem der Ansprüche 2 - 5, **dadurch gekennzeichnet, daß** die Gesamtmenge an einem oder mehreren grenzflächenaktiven Substanzen aus der Gruppe der Sorbitanmonoester gewählt wird aus dem Bereich von 0,1 - 25,0 Gew.-%, bevorzugt 0,5-15,0 Gew.-%, bezogen auf das Gesamtgewicht der Zubereitungen.

**13.** Verwendung nach einem der Ansprüche 2 - 5, **dadurch gekennzeichnet, daß** die Gesamtmenge an einem oder mehreren Fettalkoholen in den fertigen kosmetischen oder dermatologischen Zubereitungen gewählt wird aus dem Bereich von 0,1 - 10,0 Gew.-%, bevorzugt 0,5 - 6,0 Gew.-%, bezogen auf das Gesamtgewicht der Zubereitungen.

**14.** Verwendung nach einem der Ansprüche 2 - 5, **dadurch gekennzeichnet, daß** Gewichtsverhältnisse von partiell neutralisierten Estern von Monoglyceriden und/oder Diglyceriden gesättigter Speisefettsäuren mit Zitronensäure und Sorbitanestern einerseits und einen oder mehrere Fettalkohole (gewählt aus der Gruppe der verzweigten und unverzweigten Alkylalkohole mit 12 bis 40 Kohlenstoffatomen) andererseits von 7 : 3 bis 3 : 7 gewählt werden, bevorzugt von 2:1 1 bis 1 : 2, insbesondere bevorzugt von etwa 1 : 1.

**15.** Verwendung nach einem der Ansprüche 2 - 5, **dadurch gekennzeichnet, daß** als partiell neutralisierten Estern von Monoglyceriden und/oder Diglyceriden gesättigter Speisefettsäuren mit Zitronensäure das Glycerytstearatcitrat gewählt wird.

## Claims

**1.** Cosmetic and dermatological preparations having at least one aqueous phase, comprising

(I) one or more partially neutralized esters of monoglycerides and/or diglycerides of saturated fatty acids with citric acid,
(II) one or more sorbitan monoesters, which are **characterized by** the structural formula

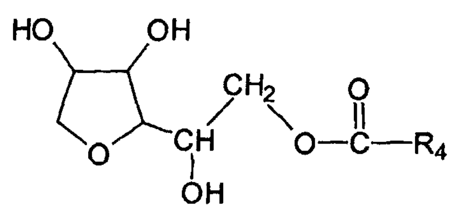

where $R_4$ is a branched or unbranched alkyl radical having 8 to 22 carbon atoms,
(III) one or more fatty alcohols, chosen from the group of branched and unbranched alkyl alcohols having 12 to 40 carbon atoms,
(IV) where at least one of the aqueous phases comprises one or more electrolytes in dissolved form.

**2.** Use of

(I) one or more partially neutralized esters of monoglycerides and/or diglycerides of saturated fatty acids with citric acid,
(II) one or more sorbitan monoesters, which are **characterized by** the structural formula

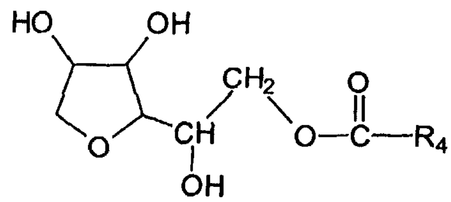

where $R_4$ is a branched or unbranched alkyl radical having 8 to 22 carbon atoms,
(III) one or more fatty alcohols, chosen from the group of branched and unbranched alkyl alcohols having 12 to 40 carbon atoms,
(IV) where at least one of the aqueous phases comprises one or more electrolytes in dissolved form,

for the preparation of cosmetic and dermatological emulsions, in particular O/W emulsions having at least one aqueous phase, for skin care.

**3.** Use of the combination of

(I) one or more partially neutralized esters of monoglycerides and/or diglycerides of saturated fatty acids with citric acid,
(II) one or more sorbitan monoesters, which are **characterized by** the structural formula

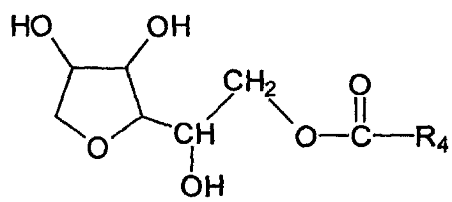

where $R_4$ is a branched or unbranched alkyl radical having 8 to 22 carbon atoms,
(III) one or more fatty alcohols, chosen from the group of branched and unbranched alkyl alcohols having 12 to 40 carbon atoms,

for achieving or increasing the stability of emulsions to the presence of electrolytes, in particular for achieving or increasing the stability of O/W emulsions to the presence of electrolytes.

**4.** Use according to Claim 3 for achieving or increasing the stability of emulsions to the presence of electrolytes, in particular for achieving or increasing the stability of O/W emulsions to the presence of electrolytes, where the emulsions, in at least one of the aqueous phases, contains [sic] one or more electrolytes in dissolved form, and the concentration of the substances dissociated into ions in the aqueous phase(s) in which the electrolyte(s) is/are present in dissolved form is at least 0.05 mol/l, in particular at least 0.075 mol/l, particularly preferably at least 0.10 mol/l.

**5.** Use according to Claim 3 for achieving or increasing the stability of emulsions to the presence of electrolytes, in particular for achieving or increasing the stability of O/W emulsions to the presence of electrolytes, where the emulsions, in at least one of the aqueous phases, contains [sic] one or more electrolytes in dissolved form, and the ionic strength of the aqueous phases in which the electrolyte(s) is/are present in dissolved form is at least 0.05 mol/l, in particular at least 0.075 mol/l, particularly preferably at least 0.10 mol/l.

**6.** Preparation according to Claim 1, **characterized in that** the total amount of one or more partially neutralized esters of monoglycerides and/or diglycerides of saturated fatty acids with citric acid in the finished cosmetic or dermatological preparations is chosen from the range 0.1 - 10.0% by weight, preferably 0.5 - 6.0% by weight, based on the total weight of the preparations.

**7.** Preparation according to Claim 1, **characterized in that** the total amount of one or more interface-active substances from the group of sorbitan monoesters is chosen from the range 0.1 - 25.0% by weight, preferably 0.5 -

15.0% by weight, based on the total weight of the preparations.

8. Preparation according to Claim 1, **characterized in that** the total amount of one or more fatty alcohols in the finished cosmetic or dermatological preparations is chosen from the range 0.1 - 10.0% by weight, preferably 0.5 - 6.0% by weight, based on the total weight of the preparations.

9. Preparation according to Claim 1, **characterized in that** weight ratios of partially neturalized esters of monoglycerides and/or diglycerides of saturated food fatty acids with citric acid and sorbitan esters on the one hand and one or more fatty alcohols (chosen from the group of branched and unbranched alkyl alcohols having 12 to 40 carbon atoms) on the other hand of from 7:3 to 3:7 are chosen, preferably from 2:1 to 1:2, particularly preferably of about 1:1.

10. Preparation according to Claim 1, **characterized in that** the partially neutralized ester of monoglycerides and/or diglycerides of saturated food fatty acids with citric acid chosen is glyceryl stearate citrate.

11. Use according to one of Claims 2-5, **characterized in that** the total amount of one or more partially neutralized esters of monoglycerides and/or diglycerides of saturated fatty acids with citric acid in the finished cosmetic or dermatological preparations is chosen from the range 0.1 - 10.0% by weight, preferably 0.5 - 6.0% by weight, based on the total weight of the preparations.

12. Use according to one of Claims 2-5, **characterized in that** the total amount of one or more interface-active substances from the group of sorbitan monoesters is chosen from the range 0.1 - 25.0% by weight, preferably 0.5 - 15.0% by weight, based on the total weight of the preparations.

13. Use according to one of Claims 2-5, **characterized in that** the total amount of one or more fatty alcohols in the finished cosmetic or dermatological preparations is chosen from the range 0.1 - 10.0% by weight, preferably 0.5 - 6.0% by weight, based on the total weight of the preparations.

14. Use according to one of Claims 2-5, **characterized in that** weight ratios of partially neturalized esters of monoglycerides and/or diglycerides of saturated food fatty acids with citric acid and sorbitan esters on the one hand and one or more fatty alcohols (chosen from the group of branched and unbranched alkyl alcohols having 12 to 40 carbon atoms) on the other hand of from 7:3 to 3:7 are chosen, preferably from 2:1 to 1:2, particularly preferably of about 1:1.

15. Use according to one of Claims 2-5, **characterized in that** the partially neutralized ester of monoglycerides and/or diglycerides of saturated food fatty acids with citric acid chosen is glyceryl stearate citrate.

**Revendications**

1. Préparations cosmétiques et dermatologiques comportant au moins une phase aqueuse contenant

(I) un ou plusieurs esters partiellement neutralisés de monoglycérides et/ou diglycérides d'acides gras saturés avec l'acide citrique,
(II) un ou plusieurs monoesters de sorbitanne qui se distinguent par la formule développée

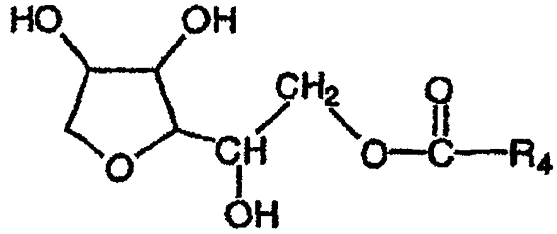

$R_4$ représentant un radical alkyle ramifié ou non ramifié ayant de 8 à 22 atomes de carbone,
(III) un ou plusieurs alcools gras, choisis dans le groupe des alcools alkyliques ramifiés et des alcools alkyliques

non ramifiés ayant de 12 à 40 atomes de carbone,
(IV) au moins l'une des phases aqueuses contenant un ou plusieurs électrolytes.

**2.** Utilisation de

(I) un ou plusieurs esters partiellement neutralisés de monoglycérides et/ou diglycérides d'acides gras saturés avec l'acide citrique,
(II) un ou plusieurs monoesters de sorbitanne qui se distinguent par la formule développée

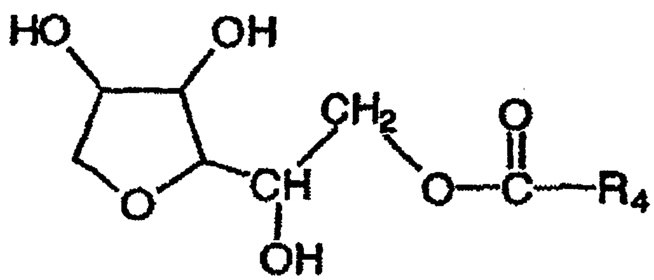

$R_4$ représentant un radical alkyle ramifié ou non ramifié ayant de 8 à 22 atomes de carbone,
(III) un ou plusieurs alcools gras, choisis dans le groupe des alcools alkyliques ramifiés et des alcools alkyliques non ramifiés ayant de 12 à 40 atomes de carbone,
(IV) au moins l'une des phases aqueuses contenant un ou plusieurs électrolytes, pour la fabrication d'émulsions cosmétiques et/ou dermatologiques, en particulier d'émulsions H/E comportant au moins une phase aqueuse, pour le soin de la peau.

**3.** Utilisation de l'association de

(I) un ou plusieurs esters partiellement neutralisés de monoglycérides et/ou diglycérides d'acides gras saturés avec l'acide citrique,
(II) un ou plusieurs monoesters de sorbitanne qui se distinguent par la formule développée

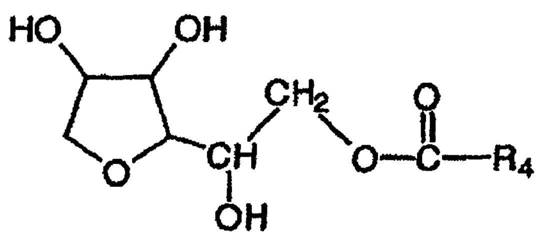

$R_4$ représentant un radical alkyle ramifié ou non ramifié ayant de 8 à 22 atomes de carbone,
(III) un ou plusieurs alcools gras, choisis dans le groupe des alcools alkyliques ramifiés et des alcools alkyliques non ramifiés ayant de 12 à 40 atomes de carbone,

pour atteindre ou augmenter la stabilité d'émulsions vis-à-vis de la présence d'électrolytes, en particulier pour atteindre ou augmenter la stabilité d'émulsions H/E vis-à-vis de la présence d'électrolytes.

**4.** Utilisation selon la revendication 3, pour atteindre ou augmenter la stabilité d'émulsions vis-à-vis de la présence d'électrolytes, en particulier pour atteindre ou augmenter la stabilité d'émulsions H/E vis-à-vis de la présence d'électrolytes, les émulsions contenant dissous dans au moins l'une des phases aqueuses un ou plusieurs électrolytes, et la concentration des substances dissociées en ions dans la ou les phases aqueuses dans lesquelles l'électrolyte ou les électrolytes sont présents à l'état dissous, étant d'au moins 0,05 mole/l, en particulier d'au moins 0,075 mole/l, de façon particulièrement préférée, d'au moins 0,10 mole/l.

5. Utilisation selon la revendication 3, pour atteindre ou augmenter la stabilité d'émulsions vis-à-vis de la présence d'électrolytes, en particulier pour atteindre ou augmenter la stabilité d'émulsions H/E vis-à-vis de la présence d'électrolytes, les émulsions contenant dissous dans au moins l'une des phases aqueuses un ou plusieurs électrolytes, et la force ionique des phases aqueuses dans lesquelles l'électrolyte ou les électrolytes sont présents à l'état dissous, étant d'au moins 0,05 mole/l, en particulier d'au moins 0,075 mole/l, de façon particulièrement préférée, d'au moins 0,10 mole/l.

6. Préparation selon la revendication 1, **caractérisée en ce que** la quantité totale d'un ou plusieurs esters partiellement neutralisés de monoglycérides et/ou diglycérides d'acides gras avec l'acide citrique dans les préparations cosmétiques ou dermatologiques finales est choisie dans la plage allant de 0,1 à 10,0 % en poids, de préférence de 0,5 à 6,0 % en poids, par rapport au poids total des préparations.

7. Préparation selon la revendication 1, **caractérisée en ce que** la quantité totale d'une ou plusieurs substances tensioactives choisies dans le groupe des monoesters de sorbitanne est choisie dans la plage allant de 0,1 à 25,0 % en poids, de préférence de 0,5 à 15,0 % en poids, par rapport au poids total des préparations.

8. Préparation selon la revendication 1, **caractérisée en ce que** la quantité totale d'un ou plusieurs alcools gras dans les préparations cosmétiques ou dermatologiques finales est choisie dans la plage allant de 0,1 à 10,0 % en poids, de préférence de 0,5 à 6,0 % en poids, par rapport au poids total des préparations.

9. Préparation selon la revendication 1, **caractérisée en ce que** les proportions pondérales d'esters partiellement neutralisés de monoglycérides et/ou diglycérides d'acides gras alimentaires saturés, avec l'acide citrique et d'esters de sorbitanne, d'une part, et d'un ou plusieurs alcools gras (choisis dans le groupe des alcools alkyliques ramifiés et des alcools alkyliques non ramifiés, ayant de 12 à 40 atomes de carbone) d'autre part, sont choisies de 7:3 à 3:7, de préférence de 2:1 à 1:2, de façon particulièrement préférée, d'environ 1:1.

10. Préparation selon la revendication 1, **caractérisée en ce qu'**on choisit en tant qu'esters partiellement neutralisés de monoglycérides et/ou diglycérides d'acides gras alimentaires saturés avec l'acide citrique le stéarate-citrate de glycéryle.

11. Utilisation selon l'une quelconque des revendications 2 à 5, **caractérisée en ce que** la quantité totale d'un ou plusieurs esters partiellement neutralisés de monoglycérides et/ou diglycérides d'acides gras alimentaires saturés avec l'acide citrique, dans les préparations cosmétiques ou dermatologiques, est choisie dans la plage allant de 0,1 à 10,0 % en poids, de préférence de 0,5 à 6,0 % en poids, par rapport au poids total des préparations.

12. Utilisation selon l'une quelconque des revendications 2 à 5, **caractérisée en ce que** la quantité totale d'une ou plusieurs substances tensioactives choisie(s) dans le groupe des monoesters de sorbitanne est choisie dans la plage allant de 0,1 à 25,0 % en poids, de préférence de 0,5 à 15,0 % en poids, par rapport au poids total des préparations.

13. Utilisation selon l'une quelconque des revendications 2 à 5, **caractérisée en ce que** la quantité totale d'un ou plusieurs alcools gras dans les préparations cosmétiques ou dermatologiques finales est choisie dans la plage allant de 0,1 à 10,0 % en poids, de préférence de 0,5 à 6,0 % en poids, par rapport au poids total des préparations.

14. Utilisation selon l'une quelconque des revendications 2 à 5, **caractérisée en ce que** les proportions pondérales d'esters partiellement neutralisés de monoglycérides et/ou diglycérides d'acides gras alimentaires saturés, avec l'acide citrique et d'esters de sorbitanne, d'une part, et d'un ou plusieurs alcools gras (choisis dans le groupe des alcools alkyliques ramifiés et des alcools alkyliques non ramifiés, ayant de 12 à 40 atomes de carbone) d'autre part, sont choisies de 7:3 à 3:7, de préférence de 2:1 à 1:2 de façon particulièrement préférée, d'environ 1:1.

15. Utilisation selon l'une quelconque des revendications 2 à 5, **caractérisée en ce qu'**on choisit en tant qu'esters partiellement neutralisés de monoglycérides et/ou diglycérides d'acides gras alimentaires saturés avec l'acide citrique le stéarate-citrate de glycéryle.